# EUROPEAN PATENT SPECIFICATION

(11) **EP 0 671 908 B1**
(45) Date of publication and mention of the grant of the patent: **06.05.1998**
(21) Application number: 93912775.9
(22) Date of filing: 24.05.1993
(51) Int. Cl.: A61K 31/00, A61K 31/445, A61K 47/06, A61K 47/12

(54) **PHARMACEUTICAL COMPOSITIONS COMPRISING 5-HT 1? RECEPTOR AGONISTS AND ABSORPTION ENHANCERS**
PHARMAZEUTISCHE ZUSAMMENSETZUNGEN, DIE 5-HT 1-REZEPTORAGONISTEN UND ABSORPTIONSVERSTÄRKER ENTHALTEN
COMPOSITIONS PHARMACEUTIQUES COMPRENANT DES AGONISTES DES RECEPTEURS 5-HT 1? ET DES STIMULATEURS D'ABSORPTION

(30) Priority: 28.05.1992 GB 9211277
(43) Date of publication of application: 20.09.1995
(73) Proprietor: GLAXO CANADA INC., Mississauga, Ontario L5N 6L4 (CA)
(72) Inventor: DOHERTY, Christopher, Priory Street , Ware Hertfordshire, SG12 (GB); GAMBHIR, Renu Glaxo Canada Inc., Mississauga, Ontario L5N 6L4 (CA)
(74) Representative: Filler, Wendy Anne, Dr.
(86) International application number: EP9301301
(87) International publication number: WO9324116

(56) References cited:
- EP-A- 0 143 949
- EP-A- 0 303 507
- GB-A- 2 162 522
- EUR. NEUROL. vol. 31, no. 5, 1991, pages 291 - 294 P.A.FOWLER ET AL. 'The clinical pharmacology, pharmacokinetics and metabolism of sumatriptan'
- CRIT.REV.THER.DRUG.CARRIER SYST. vol. 7, no. 1, 1990, pages 1 - 33 SHOZO MURANISHI 'absorption enhancers'

## Description

The present invention relates to a liquid pharmaceutical composition for oral administration containing as active ingredient a compound having selective agonist activity at 5HT₁-like receptors, in particular a composition comprising oleic or linoleic acid as an absorption enhancing carrier.

5-HT₁-like receptors are located, for example, in the dog saphenous vein and the 5-HT₁-like receptor agonists with which the present invention is concerned contract the dog saphenous vein. Such compounds may therefore be identified by their contractile effect on the dog isolated saphenous vein strip as described, for example, by Apperley et al., Br. J. Pharmacol, 68, 215-224 (1980). Compounds which are selective 5-HT₁-like receptor agonists have also been found to selectively constrict the carotid arterial bed of the anaesthetised dog.

A variety of compounds which selectively constrict the dog isolated saphenous vein strip and which constrict the carotid arterial bed of the anaesthetised dog have been described in the art. These include indole derivatives such as those disclosed inter alia in published British Patent Specifications Nos. 2082175, 2081717, 2083463, 2124210, 2150932, 2162522, 2168347, 2168973, 2185020, 2186874, 2191488, 2208646, published European Patent Specifications Nos. 147107, 237678, 242939, 244085, 225726, 254433, 303506, 313397, 354777, 382570, 464558, 506363, 506369, 450238, 451022, 451008, 478954, 438230, 494774, 497512, 501568 and published International patent application Nos. WO92/11013, W092/11014, WO92/06973, WO93/00086, WO92/13856, WO93/00094, WO91/18897 and WO93/00333. The compounds disclosed in the specifications (hereinafter described as compounds A) are useful in the treatment of migraine and cluster headache.

EP-A-0303507 discloses a class of piperidinyl substituted indole derivatives which display 5HT₁-like receptor agonist activity. It also discloses certain pharmaceutical formulations which contain at least one such derivative as active ingredient. N-methyl-3-(1-methyl-4-piperidinyl)-1H-indole-5-ethanesulphonamide is described as being a preferred compound.

EP-A-143949 describes specific pharmaceutical compositions containing urokinase and a physiological absorption enhancer comprising a combination of higher fatty acids, a polyalkylene glycol and calcium. The compositions may be administered orally.

Eur. Neurol., 1991 vol 31 pages 291-294 describes studies of sumatriptan pharmacokinetics following oral and subcutaneous administration in a small number of volunteers and migraine sufferers. It states that the comparatively lower bioavailability of orally administered sumatriptan is mainly due to presystemic metabolism.

The present invention provides a liquid pharmaceutical composition for oral administration which comprises 5 to 30% of a compound which acts as a 5HT₁-like receptor agonist or a physiologically acceptable salt or solvate thereof, as active ingredient, and an absorption enhancing carrier which comprises oleic or linoleic acid.

Oral administration constitutes the generally preferred route for administration of pharmaceuticals since this route is particularly convenient and acceptable to patients. It is highly desirable, particularly in the treatment of acute conditions such as migraine, that pharmaceutical compositions have a rapid and consistent onset of action combined with sustained activity and good bioavailability. Rapid absorption can be achieved by parenteral injection but this is unacceptable to some patients, particularly if the drug is to be administered without direct medical supervision, i.e. self-administered. We have now found that the compositions according to the invention have excellent stability, formulation and pharmacokinetic characteristics.

In a preferred embodiment of the invention we provide a liquid pharmaceutical composition for oral administration which comprises 5 to 30% of one or more of compounds A or a pharmaceutically acceptable salt or solvate thereof, as active ingredient, and an absorption enhancing carrier which comprises oleic or linoleic acid.

Compositions according to the invention are preferably in a form adapted for use in medicine, in particular human medicine.

Particularly preferred compounds for use in the compositions of the present invention are 3-[2-(dimethylamino)ethyl]-N-methyl-1H-indole-5-methanesulphonamide and N-methyl-3-(1-methyl-4-piperidinyl)-1H-indole-5-ethanesulphonamide, especially 3-[2-(dimethylamino)ethyl]-N-methyl-1H-indole-5-methanesulphonamide.

3-[2-(Dimethylamino)ethyl]-N-methyl-1H-indole-5-methanesulphonamide, which may be represented by the formula (I) 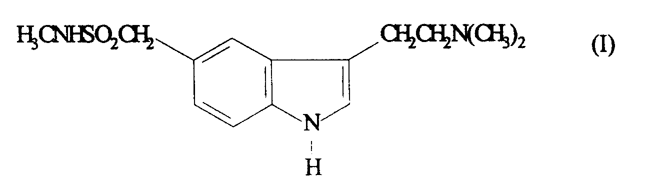 and its physiologically acceptable salts and solvates are disclosed in GB 2162522. The compound of formula (I) is described as useful in treating and/or preventing pain resulting from dilatation of the cranial vasculature, in particular migraine.

Numerous clinical studies have demonstrated the effectiveness of the compound of formula (I) (generic name sumatriptan) in migraineurs, either by oral or intranasal administration or by parenteral injection. Nevertheless, alternative oral formulations of sumatriptan with an enhanced onset of action are highly desirable.

The applicants have demonstrated that certain liquid formulations of sumatriptan provide faster absorption than solid dosage forms (i.e. tablets) of sumatriptan.

The invention thus provides in a particularly preferred aspect a liquid formulation for oral administration comprising 5 to 30% of 3-[2-(dimethylamino)ethyl]-N-methyl-1H-indole-5-methane-sulphonamide or a pharmaceutically acceptable salt or solvate thereof and an absorption enhancing carrier which comprises oleic or linoleic acid.

The liquid formulations according to the invention may be in the form of a solution, an emulsion or a suspension.

Pharmaceutically acceptable salts of compounds A include those derived from pharmaceutically acceptable inorganic and organic acids. Examples of suitable acids include hydrochloric, hydrobromic, sulphuric, nitric, perchloric, fumaric, maleic, phosphoric, glycolic, lactic, salicylic, succinic, toluene-p-sulphonic, tartaric, acetic, citric, methanesulphonic, formic, benzoic, malonic, naphthalene-2-sulphonic and benzenesulphonic acids. A preferred salt is a succinate salt, more particularly the (1:1) succinate salt. However, the applicants have found that formulations of the present invention are surprisingly advantageous when the active compound is in the form of its free base.

Absorption enhancing carriers include water immiscible liquids, solids or semi-solids such as vegetable oils, aromatic oils, aliphatic and aromatic hydrocarbons, fatty acids and their triglycerides and esters, chlorinated hydrocarbons, ethers and esters or water-miscible non-volatile liquids, solids or semi-solids such as propylene glycol and other polyethylene glycols (e.g. PEG300, 400 or 600) and nonionic or amphoteric surfactants such as lecithin. The absorption enhancing carrier in compositions according to the invention comprises oleic acid or linoleic acid, either alone or advantageously in combination with one or more further absorption enhancing carriers such as polyethylene glycol and propylene glycol. Oleic acid is a particularly preferred absorption enhancing carrier.

Preferably surface active agents, such as polysorbate, bile salts and lecithin will also be present in the formulation. A particularly preferred surface active agent is polysorbate 80.

The active compound will be present in the liquid formulation in an amount of 5 to 30% w/w, in particular from 10 to 25% w/w, preferably from 14-17% w/w.

The absorption enhancing carrier may either provide the balance of the formulation when present as the only excipient (i.e. from 95-70% w/w of the formulation) or where other excipients are present from 40-80% w/w of the formulation, for example 50-80% in particular from 60-75% of the formulation.

Surface active agents may be present in an amount of from 0.01 to 20% w/w, in particular from 1 to 20%, for example 10% w/w.

A preferred formulation according to the invention comprises 5 to 30% of a compound which acts as a 5HT₁-like receptor agonist in the form of its free base and oleic acid as absorption enhancing carrier, optionally together with a polyethylene glycol. A particularly preferred formulation further comprises polysorbate as surface active agent.

The formulations of the invention may be employed directly in liquid form for oral administration.

Conveniently, however, the formulation is presented in unit dosage form in gelatin capsules, in particular soft gelatin capsules, although hard gelatin capsules may also be employed.

Thus the invention provides in a further aspect a pharmaceutical formulation in the form of a capsule and comprising a liquid fill comprising 5 to 30% of a compound which acts as a 5HT₁-like receptor agonist or a pharmaceutically acceptable salt or solvate thereof and an absorption enhancing carrier which comprises oleic or linoleic acid, surrounded by a gelatin shell.

When the formulation is presented in the form of a capsule, the active compound will be present in an amount of from 1 to 200mg, preferably 10 to 200mg, for example 25 to 100mg of active compound calculated as the free base.

The pharmaceutical formulations of the invention may also contain other active ingredients, in particular other antimigraine agents, antinauseants or analgesics.

The formulations may be prepared in conventional manner, for example by intimate admixture of the active ingredient with the carrier. Preferably the formulations are manufactured with minimum exposure to air, for example processed under nitrogen. However, the formulations may contain antioxidants such as butylated hydroxyanisole, butylated hydroxytoluene, lactic acid and ascorbyl palmitate or preservatives in order to minimise oxidation and microbial contamination. Antioxidants may be present in an amount of from 0.005 to 0.1% w/w, preferably 0.01 to 0.05% w/w. Other excipients conventionally used in the art of pharmaceutical formulation may also be present.

A further aspect of the invention provides a method for the treatment of a mammal, including man, suffering from or susceptible to conditions associated with cephalic pain such as cluster headache, chronic paroxysmal hemicrania, headache associated with vascular disorders, headache associated with substances or their withdrawal (for example drug withdrawal), tension headache and in particular migraine which comprises oral administration of a liquid pharmaceutical composition which comprises 5 to 30% of a compound which acts as a 5HT₁-like receptor agonist or a pharmaceutically acceptable salt or solvate thereof as active ingredient, together with a pharmaceutically acceptable absorption enhancing carrier which comprises oleic or linoleic acid. It will be appreciated that reference to treatment is intended to include prophylaxis as well as the alleviation of established symptoms.

It will be appreciated that the amount of compounds which act as 5HT₁-like receptor agonists employed in the compositions of the invention will depend on the particular compounds used. Furthermore, the precise therapeutic dose of the active ingredient will depend on the age and condition of the patient and the nature of the condition to be treated and will be at the ultimate discretion of the attendant physician.

However, in general, effective doses for the treatment of conditions associated with cephalic pain, for example acute treatment of migraine, will lie in the range of 1 to 500mg, preferably 2 to 200mg, most preferably 5 to 100mg, for example 10mg or 25mg of the active ingredient per unit dose which could be administered in single or divided doses, for example, 1 to 4 times per day.

The following examples are illustrative of the invention.

### Example 1 - 7

Liquid formulations were prepared by slow addition of the active ingredient (A) into the other ingredients (B-D) at 35-50°C with constant mixing (amounts are given as percentage w/w).

| Example | 1 | 2 | 3 | 4 | 5 | 6 | 7 |
|---|---|---|---|---|---|---|---|
| A1 Sumatriptan (free base) | 16.7 | 16.7 | 16.7 | 14.3 | 14.29 | 22.30 | - |
| A2 Sumatriptan (hemisuccinate) | - | - | - | - | - | - | 30 |
| B1 Oleic Acid NF | 73.3 | 73.285 | 83.3 | 64.3 | 53.56 | 77.65 | - |
| B2 Polyethylene Glycol 4000 NF | - | - | - | - | - | - | 3 |
| B3 Polyethylene Glycol 400 NF | - | - | - | 21.4 | 21.43 | - | 67 |
| C1 Polysorbate 80 NF | 10.0 | 10.0 | - | - | 10.72 | - | - |
| C2 Lecithin | - | - | - | - | - | 0.02 | - |
| D1 Butylated Hydroxyanisole | - | - | - | - | - | 0.015 | - |
| D2 Butylated Hydroxytoluene | - | - | - | - | - | 0.015 | - |
| D3 Ascorbyl palmitate | - | 0.015 | - | - | - | - | - |

### Examples 8 and 9

Liquid formulations were prepared by slow addition of active ingredient (A) into the other ingredients (B-D) at 35-50°C with constant mixing (amounts are given as percentage w/w).

| Example | 8 | 9 |
|---|---|---|
| A1 Sumatriptan (free base) | 18.2 | 18.2 |
| B1 Oleic acid | 60.985 | 68.485 |
| B2 Polyethylene glycol 600 | 7.3 | 7.3 |
| B3 Propylene glycol | 6.0 | 6.0 |
| C1 Polysorbate 80 | 7.5 | - |
| D1 Ascorbyl palmitate | 0.015 | 0.015 |

### Examples 10-13

The liquid formulations of Examples 1, 3, 4 and 5 were filled into hard gelatin capsules, each capsule containing 25mg of sumatriptan.

### Examples 14 - 22

The liquid formulations of Examples 1 to 9 were filled into hard gelatin capsules, each capsule containing 100mg of sumatriptan.

### Examples 23 - 31

Liquid formulations are prepared as described in Examples 1 to 9 wherein the active ingredient A1 was N-methyl-3-(1-methyl-4-piperidinyl)-1H-indole-5-ethanesulphonamide and active ingredient A2 was N-methyl-3-(1-methyl-4-piperidinyl)-1H-indole-5-ethanesulphonamide hydrochloride.

### Examples 32 - 40

The liquid formulations of Examples 23 to 31 are filled into hard gelatin capsules, each capsule containing 10mg of N-methyl-3-(1-methyl-4-piperidinyl)-1H-indole-5-ethanesulphonamide.

## Claims

1. A liquid pharmaceutical composition for oral administration which comprises 5 to 30% of a compound which acts as a 5HT₁-like receptor agonist or a physiologically acceptable salt or solvate thereof, as active ingredient, and an absorption enhancing carrier which comprises oleic or linoleic acid.

2. A pharmaceutical composition as claimed in claim 1 wherein the active ingredient is selected from 3-[2-(dimethylamino)ethyl]-N-methyl-1H-indole-5-methanesulphonamide, N-methyl-3-(1-methyl-4-piperidinyl)-1H-indole-5-ethanesulphonamide and pharmaceutically acceptable salts and solvates thereof.

3. A pharmaceutical composition as claimed in claim 1 wherein the active ingredient is 3-(2-(dimethylamino)ethyl]-N-methyl-1H-indole-5-methanesulphonamide or a pharmaceutically acceptable salt or solvate thereof.

4. A pharmaceutical composition as claimed in any one of claims 1 to 3 wherein the active ingredient is in the form of its free base.

5. A pharmaceutical composition as claimed in any one of claims 1 to 4 wherein the absorption enhancing carrier comprises oleic acid.

6. A pharmaceutical composition as claimed in any one of claims 1 to 5 which further comprises a polyethylene glycol.

7. A pharmaceutical composition as claimed in any one of claims 1 to 6 which further comprises propylene glycol.

8. A pharmaceutical composition as claimed in any one of claims 1 to 7 which further comprises a surface active agent.

9. A pharmaceutical composition as claimed in claim 8 wherein the surface active agent is selected from polysorbate, bile salts and lecithin.

10. A pharmaceutical composition as claimed in claim 9 wherein the surface active agent is polysorbate 80.

11. A pharmaceutical composition as claimed in any one of claims 8 to 10 wherein the surface active agent comprises 0.01 to 20% w/w of the composition.

12. A liquid pharmaceutical composiiton for oral administration which comprises 5 to 30% w/w of a compound which acts as a 5HT₁-like receptor agonist in the form of its free base and oleic acid as absorption enhancing carrier, optionally together with a polyethylene glycol.

13. A pharmaceutical composition as claimed in claim 12 which further comprises polysorbate.

14. A pharmaceutical composition as claimed in any one of claims 1 to 13 in unit dosage form.

15. A pharmaceutical composition in the form of a gelatin capsule comprising a liquid fill of a liquid pharmaceutical composition as claimed in any one of claims 1 to 14 surrounded by a gelatin shell.

16. A pharmaceutical composition as claimed in claim 14 or claim 15 which comprises 1 to 200mg of active ingredient.

17. A method for the manufacture of a pharmaceutical composition as claimed in any one of claims 1 to 16 which comprises intimate admixture of the active ingredient with the carrier.

## Patentansprüche

1. Flüssige pharmazeutische Zusammensetzung zur oralen Verabreichung, die 5 bis 30 % einer Verbindung, die als Agonist für 5HT₁-artige Rezeptoren wirkt, oder eines physiologisch akzeptablen Salzes oder Solvats davon als Wirkstoff und einen absorptionsverstärkenden Träger umfaßt, der Olein- oder Linolsäure umfaßt.

2. Pharmazeutische Zusammensetzung gemäß Anspruch 1, worin der Wirkstoff ausgewählt ist aus 3-[2-(Dimethylamino)ethyl]-N-methyl-1H-indol-5-methansulfonamid, N-Methyl-3-(1-methyl-4-piperidinyl)-1H-indol-5-ethansulfonamid und pharmazeutisch akzeptablen Salzen und Solvaten davon.

3. Pharmazeutische Zusammensetzung gemäß Anspruch 1, worin der Wirkstoff 3-[2-(Dimethylamino)ethyl]-N-methyl-1H-indol-5-methansulfonamid oder ein pharmazeutisch akzeptables Salz oder Solvat davon ist.

4. Pharmazeutische Zusammensetzung gemäß einem der Ansprüche 1 bis 3, worin der Wirkstoff in Form seiner freien Base ist.

5. Pharmazeutische Zusammensetzung gemäß einem der Ansprüche 1 bis 4, worin der absorptionsverstärkende Träger Oleinsäure umfaßt.

6. Pharmazeutische Zusammensetzung gemäß einem der Ansprüche 1 bis 5, die zusüätzlich ein Polyethylenglykol umfaßt.

7. Pharmazeutische Zusammensetzung gemäß einem der Ansprüche 1 bis 6, die zusätzlich Propylenglykol umfaßt.

8. Pharmazeutische Zusammensetzung gemäß einem der Ansprüche 1 bis 7, die zusätzlich ein Tensid umfaßt.

9. Pharmazeutische Zusammensetzung gemäß Anspruch 8, worin das Tensid ausgewählt ist aus Polysorbat, Gallensalzen und Lecithin.

10. Pharmazeutische Zusammensetzung gemäß Anspruch 9, worin das Tensid Polysorbat 80 ist.

11. Pharmazeutische Zusammensetzung gemäß einem der Ansprüche 8 bis 10, worin das Tensid 0,01 bis 20 % G/G in der Zusammensetzung umfaßt.

12. Flüssige pharmazeutische Zusammensetzung zur oralen Verabreichung, die 5 bis 30 % G/G einer Verbindung, die als Agonist für 5HT₁-artige Rezeptoren wirkt, in Form ihrer freien Base und Oleinsäure als absorptionsverstärkenden Träger, gegebenenfalls zusammen mit einem Polyethylenglykol, umfaßt.

13. Pharmazeutische Zusammensetzung gemäß Anspruch 12, die zusätzlich Polysorbat umfaßt.

14. Pharmazeutische Zusammensetzung gemäß einem der Ansprüche 1 bis 13 in Einheitsarzneiform.

15. Pharmazeutische Zusammensetzung in Form einer Gelatinekapsel, umfassend eine flüssige Füllung einer flüssigen pharmazeutischen Zusammensetzung gemäß einem der Ansprüche 1 bis 14, die von einer Gelatinehülle umgeben ist.

16. Pharmazeutische Zusammensetzung gemäß Anspruch 14 oder 15, die 1 bis 200 mg des Wirkstoffs umfaßt.

17. Verfahren zur Herstellung einer pharmazeutischen Zusammensetzung gemäß einem der Ansprüche 1 bis 16, welches das innige Vermischen des Wirkstoffs mit dem Träger umfaßt.

## Revendications

1. Composition pharmaceutique liquide pour administration par voie orale qui comprend de 5 à 30% d'un composé qui agit comme un agoniste d'un récepteur de type 5HT₁ ou un sel ou solvate physiologiquement acceptable de celui-ci, à titre de principe actif, et un véhicule stimulant l'absorption qui comprend de l'acide oléique ou de I'acide linoléique.

2. Composition pharmaceutique selon la revendication 1, dans laquelle le principe actif est choisi parmi le 3-[2-(diméthylamino)éthyl]-N-méthyl-1H-indole-5-méthanesulfonamide, le N-méthyl-3-(1-méthyl-4-pipéridinyl)1-H-indole-5-éthanesulfonamide et des sels et solvates pharmaceutiquement acceptables de ceux-ci.

3. Composition pharmaceutique selon la revendication 1, dans laquelle le principe actif est le 3-[2-(diméthylamino)éthyl]-N-méthyl-1H-indole-5-méthanesulfonamide ou un sel ou solvate pharmaceutiquement acceptable de celui-ci.

4. Composition pharmaceutique selon l'une quelconque des revendications 1 à 3, dans laquelle le principe actif est sous forme de la base libre correspondante.

5. Composition pharmaceutique selon l'une quelconque des revendications 1 à 7, dans laquelle le véhicule stimulant l'absorption comprend de l'acide oléique.

6. Composition pharmaceutique selon l'une quelconque des revendications 1 à 5 qui comprend en outre un polyéthylène-glycol.

7. Composition pharmaceutique selon l'une quelconque des revendications 1 à 6 qui comprend en outre du propylène-glycol.

8. Composition pharmaceutique selon l'une quelconque des revendications 1 à 7, qui comprend en outre un agent tensio-actif.

9. Composition pharmaceutique selon la revendication 8, dans laquelle l'agent tensio-actif est choisi parmi le polysorbate, les sels biliaires et la lécithine.

10. Composition pharmaceutique selon la revendication 9, dans laquelle l'agent tensio-actif est le polysorbate 80.

11. Composition pharmaceutique selon l'une quelconque des revendications 8 à 10, dans laquelle l'agent tensio-actif constitue de 0,01 à 20% en poids/poids de la composition.

12. Composition pharmaceutique liquide pour administration par voie orale qui comprend de 5 à 30% en poids[poids d'un composé qui agit comme un agoniste d'un récepteur de type 5HT₁ sous forme de la base libre correspondante et de l'acide oléique comme véhicule stimulant l'absorption, éventuellement en conjonction avec un polyéthylène-glycol.

13. Composition pharmaceutique selon la revendication 12, qui comprend en outre du polysorbate.

14. Composition pharmaceutique selon l'une quelconque des revendications 1 à 13 en présentation unitaire.

15. Composition pharmaceutique en forme d'une capsule en gélatine comprenant une charge liquide d'une composition pharmaceutique liquide selon l'une quelconque des revendications 1 à 14 entourée d'une enveloppe en gélatine.

16. Composition pharmaceutique selon la revendication 14 ou la revendication 15 qui comprend de 1 à 200 mg de principe actif.

17. Procédé pour l'obtention d'une composition pharmaceutique selon l'une quelconque des revendications 1 à 16 qui comprend le mélange uniforme du principe actif avec le véhicule.
